# EUROPEAN PATENT SPECIFICATION

(11) **EP 3 478 713 B1**
(45) Date of publication and mention of the grant of the patent: **11.05.2022**
(21) Application number: 17740200.5
(22) Date of filing: 30.06.2017
(51) Int. Cl.: C07K 16/18, A61K 39/395, A61P 25/02

(54) **COMPOSITIONS FOR TREATING AMYLOIDOSIS**
ZUSAMMENSETZUNGEN ZUR BEHANDLUNG VON AMYLOIDOSE
COMPOSITIONS DESTINÉES AU TRAITEMENT DE L'AMYLOSE

(30) Priority: 30.06.2016 US 201662357151 P
(43) Date of publication of application: 08.05.2019
(73) Proprietor: Prothena Biosciences Limited, Dublin 2, D02 VK60 (IE)
(72) Inventor: KINNEY, Gene G., Burlingame, California 94010 (US); GUTHRIE, Spencer D., San Francisco, California 94118 (US); KOLLER, Martin, Rancho Santa Fe, California 92067 (US)
(74) Representative: J A Kemp LLP
(86) International application number: PCT/US2017/040289
(87) International publication number: WO 2018/005967

(56) References cited:
- WO-A2-2009/086539
- MORIE A. GERTZ ET AL: "First-in-Human Phase I/II Study of NEOD001 in Patients With Light Chain Amyloidosis and Persistent Organ Dysfunction", JOURNAL OF CLINICAL ONCOLOGY, vol. 34, no. 10, 1 April 2016 (2016-04-01) , pages 1097-1103, XP055418883, US ISSN: 0732-183X, DOI: 10.1200/JCO.2015.63.6530
- DATABASE BIOSIS [Online] BIOSCIENCES INFORMATION SERVICE, PHILADELPHIA, PA, US; December 2015 (2015-12), ZAGO WAGNER ET AL: "NEOD001 Specifically Binds Aggregated Light Chain Infiltrates in Multiple Organs from Patients with AL Amyloidosis and Promotes Phagocytic Clearance of AL Aggregates in Vitro", XP002775104, Database accession no. PREV201600269090 & ZAGO WAGNER ET AL: "NEOD001 Specifically Binds Aggregated Light Chain Infiltrates in Multiple Organs from Patients with AL Amyloidosis and Promotes Phagocytic Clearance of AL Aggregates in Vitro", BLOOD, vol. 126, no. 23, December 2015 (2015-12), & 57TH ANNUAL MEETING OF THE AMERICAN-SOCIETY-OF-HEMATOLOGY; ORLANDO, FL, USA; DECEMBER 05 -08, 2015 ISSN: 0006-4971(print)
- DATABASE BIOSIS [Online] BIOSCIENCES INFORMATION SERVICE, PHILADELPHIA, PA, US; 2 December 2016 (2016-12-02), LIEDTKE MICHAELA ET AL: "Organ Biomarker Responses in Patients with Light Chain Amyloidosis Treated with NEOD001 Are Independent of Previous Hematologic Response", XP002775102, Database accession no. PREV201700298550 & LIEDTKE MICHAELA ET AL: "Organ Biomarker Responses in Patients with Light Chain Amyloidosis Treated with NEOD001 Are Independent of Previous Hematologic Response", BLOOD, vol. 128, no. 22, 2 December 2016 (2016-12-02), page 647, & 58TH ANNUAL MEETING AND EXPOSITION OF THE AMERICAN-SOCIETY-OF-HEMATOLOGY; SAN DIEGO, CA, USA; DECEMBER 03 -06, 2016 ISSN: 0006-4971(print)
- DATABASE BIOSIS [Online] BIOSCIENCES INFORMATION SERVICE, PHILADELPHIA, PA, US; 2 December 2016 (2016-12-02), GERTZ MORIE A ET AL: "NEOD001 Demonstrates Organ Biomarker Responses in Patients with Light Chain Amyloidosis and Persistent Organ Dysfunction: Results from the Expansion Cohort of a Phase 1/2 Study", XP002775103, Database accession no. PREV201700298547

## Description

### TECHNICAL FIELD

The present disclosure relates to the technical fields of immunology and medicine.

### BACKGROUND OF THE INVENTION

AL amyloidosis or primary amyloidosis, involves a hematological disorder caused by clonal plasma cells that produce misfolded immunoglobulin light chains. Overproduction of misfolded light chain by plasma cells results in deposits of abnormal AL protein (amyloid) in the tissues and organs of individuals with AL amyloidosis. Clinical features of AL amyloidosis include a constellation of symptoms and organ dysfunction that can include cardiac, renal, and hepatic dysfunction, gastrointestinal involvement, neuropathies and macroglossia. The mechanisms by which amyloidogenic immunoglobulin light chains result in organ dysfunction are not well characterized, however, it is hypothesized that both amyloid deposits and prefibrillar aggregates may contribute to cytotoxic effects on organs observed in patients with AL amyloidosis. AL amyloidosis is a disease entity of its own, although AL amyloidosis can occur concurrently in a small subset (up to 15%) of patients with multiple myeloma.

AL amyloidosis is a rare disorder with an estimated incidence of 8 in 1,000,000 people. Only 1200 to 3200 new cases of AL amyloidosis are reported each year in the United States. Two thirds of patients with AL amyloidosis are male and less than 5% of patients are under 40 years of age. Both the causes and origins of AL amyloidosis remain poorly understood.

Current treatment of patients with AL amyloidosis is aimed at reducing or eliminating the bone marrow disorder, *i.e.* the plasma cells that are responsible for producing the light chains, thereby limiting or halting the production of amyloid. The most aggressive treatment options include stem cell transplant and high-dose chemotherapy for those patients who can tolerate it. Other treatment regimens include combinations of drugs often used to treat hematological malignancies, such as melphalan, prednisone, dexamethasone and proteosome inhibitors such as bortezomib, in an attempt to reduce light chain production. There are no currently approved treatments for AL amyloidosis that directly target potentially toxic forms of the amyloidogenic proteins. While some treatment options may ameliorate some of the morbidity associated with AL amyloidosis, none have been demonstrated to improve peripheral neuropathy, restore lost neural function, or consistently achieve cardiac or renal response rates greater than 40%.

A different form of systemic amyloidosis, AA amyloidosis or secondary amyloidosis, occurs "secondarily" as a result of other illness, such as chronic inflammatory diseases (for example, rheumatoid arthritis and ankylosing spondylitis) or chronic infections (for example, tuberculosis or osteomyelitis). In secondary amyloidosis, the depositing amyloid protein is amyloid A protein, derived from an acute-phase protein serum amyloid A. The treatment of secondary amyloidosis is directed at treating the underlying illness.

Thus, there is a need for therapies that not only slow progression but that also improve cardiac, renal and/or neural function in patients with AL amyloidosis or AA amyloidosis. The present disclosure relates to the treatment of such patients with pharmaceutical formulations of 2A4 and 7D8 antibodies, and chimeric and humanized versions thereof, which show high affinity binding to AL amyloids due to a shared immunogenic epitope of the pathological forms of these proteins.

JOURNAL OF CLINICAL ONCOLOGY, vol. 34, no. 10, April 2016, pages 1097-1103 is a phase I/II study pertaining to the use of NEOD001 in amyloidosis patients primarily directed at determining optimum dosage.

### SUMMARY OF THE INVENTION

The invention to which the present invention pertains is set out in the claims appended to this description.

The detailed technical disclosure set out below may in some respects go beyond the disclosure of the invention per se, and may also provide technical background for related technical developments. It will be appreciated that the additional technical background is not intended to define the invention (which is defined exclusively by the appended claims), but rather to place it in a broader technical context. Accordingly, it will be appreciated that the term 'embodiments' reflects a specific detail of the disclosure and 'the elements of the additional technical background' are not intended to define as part of the invention aspects that do not fall within the scope of the appended claims.

The present disclosure relates to methods of improving cardiac, renal and/or neural function in patients having AL amyloidosis. In one aspect, the disclosure relates to a method of treating a patient with peripheral neuropathy associated with AL amyloidosis, comprising administering an effective dosage of a pharmaceutical formulation comprising an antibody which competes for binding to human amyloid A peptide with antibody 2A4 (ATCC Accession Number 9662), thereby improving the neuropathy in the patient.

In some aspects of the present disclosure, peripheral neuropathy in such patients is improved or reversed. In some aspects, neural function is restored in such patients. In some aspects, a greater than 30% cardiac and/or renal response rate in a population of patients diagnosed with AL amyloidosis is achieved. In some aspects, the patient may have previously received one or more treatments for AL amyloidosis. Such a patient may, or may not, have experienced cardiac and/or renal improvement as a result of such treatment. Such a patient may be administered an antibody as described herein in order to treat, retard, halt or reverse the progression of peripheral neuropathy associated with AL amyloidosis in the patient. A treatment regimen in accordance with the teachings herein may advantageously require less of the antibody to be administered to the patient than would be required to see improvements in cardiac and/or renal function (if any).

The present disclosure also relates to an antibody for use in methods of improving cardiac, renal and/or neural function in patients having AL amyloidosis. In one aspect, the disclosure relates to an antibody which competes for binding to human amyloid A peptide with antibody 2A4 (ATCC Accession Number 9662) for use in a method of treating a patient with peripheral neuropathy associated with AL amyloidosis.

The present disclosure also relates to the use of an antibody in a method of improving cardiac, renal and/or neural function in patients having AL amyloidosis. In one aspect, the disclosure relates to the use of an antibody which competes for binding to human amyloid A peptide with antibody 2A4 (ATCC Accession Number 9662) in a method of treating a patient with peripheral neuropathy associated with AL amyloidosis.

The present disclosure also relates to the use of an antibody in the manufacture of a medicament for improving cardiac, renal and/or neural function in patients having AL amyloidosis. In one aspect, the disclosure relates to the use of an antibody which competes for binding to human amyloid A peptide with antibody 2A4 (ATCC Accession Number 9662) in the manufacture of a medicament for treating a patient with peripheral neuropathy associated with AL amyloidosis.

The present disclosure also relates to a method of independently treating peripheral neuropathy in a patient with AL amyloidosis, comprising administering an antibody which competes for binding to human amyloid A peptide with antibody 2A4 (ATCC Accession Number 9662), wherein (a) the patient presents with peripheral neuropathy; (b) the patient has not shown any cardiac response to such dosage when previously administered; (c) the patient has not shown any renal response to such dosage when previously administered; (d) the patient previously received treatment with a different agent that did not affect the patient's peripheral neuropathy; and/or (e) the patient is receiving treatment with a different agent that does not affect the patient's peripheral neuropathy.

The present disclosure also relates to an antibody which competes for binding to human amyloid A peptide with antibody 2A4 (ATCC Accession Number 9662) in a method of independently treating peripheral neuropathy in a patient with AL amyloidosis, wherein the patient has one or more of (a)-(e) described above.

The present disclosure also relates to the use of an antibody which competes for binding to human amyloid A peptide with antibody 2A4 (ATCC Accession Number 9662) in the manufacture of a medicament for the independent treatment of a patient with peripheral neuropathy associated with AL amyloidosis, wherein the patient has one or more of (a)-(e) described above.

The present disclosure also relates to the use of an antibody which competes for binding to human amyloid A peptide with antibody 2A4 (ATCC Accession Number 9662) in the manufacture of a medicament for the independent treatment of a patient with peripheral neuropathy associated with AL amyloidosis, wherein the patient has one or more of (a)-(e) described above.

In some aspects of the present disclosure, some antibodies may specifically bind to an epitope comprising AEDS (SEQ ID NO: 18). Some antibodies comprise three CDRs of a light chain variable region and/or three CDRs of a heavy chain variable region of antibody 2A4 or 7D8. For example, the antibody comprises a light chain variable region and/or a heavy chain variable region of antibody 2A4 or 7D8 or the antibody is a chimeric or humanized version of antibody 2A4 or 7D8 or an antigen-binding fragment thereof.

In some aspects of the present disclosure, the antibody is formulated as and/or administered as a pharmaceutical formulation that not only comprises the antibody, but also comprises a histidine buffer, trehalose, polysorbate 20 and may be formulated within a particular pH range. In some aspects of the disclosure, the pharmaceutical formulation is administered intravenously or subcutaneously to the patient at particular time intervals and dosages. Such time intervals and dosages may be predetermined and/or may be adjusted based on measurable improvements in neural function or other indicia of peripheral neuropathy (e.g., NIS-LL).

### BRIEF DESCRIPTION OF THE DRAWINGS

Figures 1A-1B show the cardiac functional biomarker response in AL amyloidosis patients treated with NEOD001.
Figures 2A-2B show the renal functional biomarker response in AL amyloidosis patients treated with NEOD001.
Figure 3 shows the peripheral neuropathy progression in ATTR amyloidosis patients treated with Tafamidis or Diflunisal.
Figures 4A-4B show the point change and percent change, respectively, in NIS-LL of patients treated with NEOD001.
Figure 5 shows the peripheral neuropathy response in AL amyloidosis patients treated with NEOD001.

### DETAILED DESCRIPTION OF THE INVENTION

The present disclosure relates to improving cardiac, renal and/or neural function in patients having AL amyloidosis. Some such aspects of the disclosure relate to improving peripheral neuropathy in such patients. Some aspects of the disclosure relate to reversing the progression of peripheral neuropathy and some aspects of the disclosure relate to restoring some neural function in such patients. Some aspects of the disclosure relate to achieving a greater than 30% cardiac and/or renal response rate in a population of patients diagnosed with AL amyloidosis. Some patients have not shown a neuropathy response but have shown a cardiac and/or renal response greater than 30%, for example greater than 40%.

Some aspects of the disclosure relate to a method of treating a patient with peripheral neuropathy associated with AL amyloidosis, comprising administering an antibody which competes for binding to human amyloid A peptide with antibody 2A4 (ATCC Accession Number 9662), thereby improving the neuropathy in the patient.

Some aspects of the disclosure relate to an antibody which competes for binding to human amyloid A peptide with antibody 2A4 (ATCC Accession Number 9662) or antibody 7D8 (ATCC Accession Number PTA-9468) for use in methods of improving cardiac, renal and/or neural function in patients having AL amyloidosis.
Some aspects of the disclosure relate to the use of an antibody which competes for binding to human amyloid A peptide with antibody 2A4 (ATCC Accession Number 9662) in a method of treating a patient with peripheral neuropathy associated with AL amyloidosis.

Some aspects of the disclosure relate to the use of an antibody which competes for binding to human amyloid A peptide with antibody 2A4 (ATCC Accession Number 9662) in the manufacture of a medicament for the treatment of a patient with peripheral neuropathy associated with AL amyloidosis.

Some aspects of the disclosure relate to a method of independently treating peripheral neuropathy in a patient with AL amyloidosis, comprising administering an antibody which competes for binding to human amyloid A peptide with antibody 2A4 (ATCC Accession Number 9662), wherein (a) the patient presents with peripheral neuropathy; (b) the patient has not shown any cardiac response to such dosage when previously administered; (c) the patient has not shown any renal response to such dosage when previously administered; (d) the patient previously received treatment with a different agent that did not affect the patient's peripheral neuropathy; and/or (e) the patient is receiving treatment with a different agent that does not affect the patient's peripheral neuropathy.

Some aspects of the disclosure relate to an antibody which competes for binding to human amyloid A peptide with antibody 2A4 (ATCC Accession Number 9662) in a method of independently treating peripheral neuropathy in a patient with AL amyloidosis, wherein the patient has one or more of (a)-(e) described above.

Some aspects of the disclosure relate to the use of an antibody which competes for binding to human amyloid A peptide with antibody 2A4 (ATCC Accession Number 9662) in a method of independent treatment of a patient with peripheral neuropathy associated with AL amyloidosis, wherein the patient has one or more of (a)-(e) described above.

Some aspects of the disclosure relate to the use of an antibody which competes for binding to human amyloid A peptide with antibody 2A4 (ATCC Accession Number 9662) in the manufacture of a medicament for the independent treatment of a patient with peripheral neuropathy associated with AL amyloidosis, wherein the patient has one or more of (a)-(e) described above.

Some aspects of the disclosure relate to particular antibodies or antigen-binding fragments thereof. In some aspects, the antibody is a humanized version of 2A4. In some aspects, the antibody comprises a light chain variable region comprising three complementarity determining regions set forth as SEQ ID NOs: 6, 7 and 8, and a heavy chain variable region comprising three complementarity determining regions set forth as SEQ ID NOs: 9, 10 and 11. In some aspects, the light chain variable region comprises the amino acid sequence set forth as SEQ ID NO: 4. In some aspects, wherein the heavy chain variable region comprises the amino acid sequence set forth as SEQ ID NO: 5. In some aspects, the light chain variable region comprises the amino acid sequence set forth as SEQ ID NO: 4 and the heavy chain variable region comprises the amino acid sequence set forth as SEQ ID NO: 5. In some aspects, the antibody is a Fab, Fab', F(ab')₂, F(ab)c, Dab, nanobody or Fv.

Some aspects of the disclosure relate to pharmaceutical formulations, e.g., pharmaceutical formulations comprising a) the antibody at a concentration within the range from about 1 mg/mL to about 100 mg/mL; b) histidine buffer at a concentration within the range from about 20 mM to about 30 mM; c) trehalose at a concentration within the range from about 210 mM to about 250 mM; and d) polysorbate 20 at a concentration within the range from about 0.005% to about 0.05% by weight; and the formulation is characterized by a pH within the range from about 6 to about 7. In some aspects, a) the antibody is present at a concentration of about 50 mg/mL; b) the histidine buffer is present at a concentration of about 25 mM; c) the trehalose is present at a concentration of about 230 mM; d) the polysorbate 20 is present at a concentration of about 0.2 g/L; and the pH is about 6.5.

Some aspects of the disclosure relate to dosage and treatment regimens. In some aspects, the dosage is from about 0.5 mg/kg to about 30 mg/kg and the antibody is administered intravenously or subcutaneously at a frequency of from about weekly to about quarterly. In some aspects, the dosage is about 24 mg/kg and the antibody is administered intravenously every 28 days. In some aspects, the duration of the treatment is at least 9 months. In some aspects, the duration of the treatment is at least 12 months. In some aspects, the duration of treatment is effective to achieve or maintain less than a 2-point increase in NIS-LL from baseline. In some aspects, the duration is effective to achieve or maintain at least a 10% decrease in NIS-LL from baseline. In some aspects, the duration is effective to achieve or maintain at least a 23% decrease in NIS-LL from baseline. In some aspects, the duration is effective to achieve or maintain at least a 35% decrease in NIS-LL from baseline. In some aspects, the duration is effective to achieve or maintain at least a 50% decrease in NIS-LL from baseline. In some aspects, the duration is effective to achieve or maintain at least a 75% decrease in NIS-LL from baseline. In some aspects, the duration is effective to achieve or maintain at least a 30% and 300 pg/mL decrease in NT-proBNP.

In some aspects of the disclosure, the patient previously received treatment with a different agent, e.g., melphalan, prednisone, dexamethasone, bortezomib, cyclophosphamide, lenalidomide, doxorubicin or a combination thereof. In some aspects, the patient previously received treatment with CRD, PomDex, CyBorD, BMDex, MDex, LDex, CLD or bortezomib. In some aspects, the patient received treatment with an autologous transplant. In some aspects, the patient presented with a symptom other than peripheral neuropathy. In some aspects, the patient presented with peripheral neuropathy. In some aspects, the patient has not shown any cardiac or renal response to such dosage when previously administered.

### I. Definitions

The term "antibody" includes intact antibodies and antigen-binding fragments thereof. Typically, fragments compete with the intact antibody from which they were derived for specific binding to the target including separate heavy chains, light chains Fab, Fab', F(ab')₂, F(ab)c, Dabs, nanobodies, and Fv. Fragments can be produced by recombinant DNA techniques, or by enzymatic or chemical separation of intact immunoglobulins. The term "antibody" also includes a bispecific antibody and/or a humanized antibody. A bispecific or bifunctional antibody is an artificial hybrid antibody having two different heavy/light chain pairs and two different binding sites (*see, e.g.,* Songsivilai and Lachmann, Clin. Exp. Immunol., 79:315-321 (1990); Kostelny et al., J. Immunol., 148:1547-53 (1992)).

The term "humanized immunoglobulin" or "humanized antibody" refers to an immunoglobulin or antibody that includes at least one humanized immunoglobulin or antibody chain *(i.e.,* at least one humanized light or heavy chain). The term "humanized immunoglobulin chain" or "humanized antibody chain" *(i.e.,* a "humanized immunoglobulin light chain" or "humanized immunoglobulin heavy chain") refers to an immunoglobulin or antibody chain (*i.e.,* a light or heavy chain, respectively) having a variable region that includes a variable framework region substantially from a human immunoglobulin or antibody and complementarity determining regions (CDRs) *(e.g.,* at least one CDR, preferably two CDRs, more preferably three CDRs) substantially from a non-human immunoglobulin or antibody, and further includes constant regions *(e.g.,* at least one constant region or portion thereof, in the case of a light chain, and preferably three constant regions in the case of a heavy chain). The term "humanized variable region" (e.g., "humanized light chain variable region" or "humanized heavy chain variable region") refers to a variable region that includes a variable framework region substantially from a human immunoglobulin or antibody and complementarity determining regions (CDRs) substantially from a non-human immunoglobulin or antibody.

The phrase "substantially from a human immunoglobulin or antibody" or "substantially human" means that, when aligned to a human immunoglobulin or antibody amino sequence for comparison purposes, the region shares at least 80-90%, preferably 90-95%, more preferably 95-99% identity *(i.e.,* local sequence identity) with the human framework or constant region sequence, allowing, for example, for conservative substitutions, consensus sequence substitutions, germline substitutions, backmutations, and the like. The introduction of conservative substitutions, consensus sequence substitutions, germline substitutions, backmutations, and the like, is often referred to as "optimization" of a humanized antibody or chain. The phrase "substantially from a non-human immunoglobulin or antibody" or "substantially non-human" means having an immunoglobulin or antibody sequence at least 80-95%, preferably 90-95%, more preferably, 96%, 97%, 98%, or 99% identical to that of a nonhuman organism, e.g., a non-human mammal.

Accordingly, all regions or residues of a humanized immunoglobulin or antibody, or of a humanized immunoglobulin or antibody chain, except possibly the CDRs, are substantially identical to the corresponding regions or residues of one or more native human immunoglobulin sequences. The term "corresponding region" or "corresponding residue" refers to a region or residue on a second amino acid or nucleotide sequence which occupies the same *(i.e.,* equivalent) position as a region or residue on a first amino acid or nucleotide sequence, when the first and second sequences are optimally aligned for comparison purposes.

### II. Methods of Treatment and Amenable Subjects

Provided herein are methods of treating a human patient showing symptoms of or diagnosed with AL or AA amyloidosis, comprising administering to the patient a regime of any of the formulations described herein effective to achieve greater than a 40% cardiac and/or renal response rate and/or an improvement in peripheral neuropathy (as distinguished from reduced progression).

Subjects or patients amenable to treatment using the disclosed antibody formulations include patients presently showing symptoms of amyloid disease. For example, the present methods are especially useful for individuals who have AL amyloidosis characterized by the presence of amyloid light chain-type protein fibrils. Some patients have systemic organ dysfunction attributed to AL amyloidosis, including dysfunction of the heart, kidney, liver, peripheral nervous system, gastrointestinal system, autonomic nervous system, lung, and/or soft tissue or lymphatic system. For AL amyloidosis patients having peripheral neuropathy, the formulations can be administered to improve neural function. In some such patients their cardiac or renal function is not affected by the treatment.

Some patients present with peripheral neuropathy (Rajkumar et al., Am J Med. 1998; 104(3):232-237). Prior to the methods and uses described herein, neuropathy in patients with primary systemic amyloidosis did not improve substantially with therapy, if at all. Provided herein are methods for treating such patients, comprising administering an antibody that competes for binding to human amyloid A peptide (SEQ ID NO: 2) with antibody 2A4 (ATCC Accession Number 9662), or antibody 7D8 (ATCC Accession Number PTA-9468) or an antibody that binds the same epitope of immunoglobulin light chain as antibody 2A4 or 7D8, such as any of the antibodies specifically disclosed herein. Some patients have presented with symptoms other than peripheral neuropathy, have previously been treated with the antibodies described herein, and may, or may not, have shown any cardiac response or renal response to such treatment. Some patients have shown a neuropathy response and a cardiac or a renal response, some patients have shown a neuropathy response and a cardiac response, but not a renal response, and some patients have shown a neuropathy response and renal response, but not a cardiac response. Some patients have previously received one or more treatments for AL amyloidosis using a therapeutic agent (*e.g*., melphalan, prednisone, dexamethasone, bortezomib, cyclophosphamide, lenalidomide, doxorubicin), combination regimen (e.g., CRD, PomDex, CyBorD, BMDex, MDex, LDex, CLD), autologous transplant, or a combination thereof. Such a patient may, or may not, have experienced cardiac and/or renal improvement as a result of such treatment. For some patients, treatment of a patient having AL amyloidosis with one or more approved therapeutic agents, antibody autologous transplant, or a combination thereof may be contraindicated. For example, a clinician would expect that the deleterious effects of a particular treatment or dosage regimen required to produce improvements in cardiac and/or renal function in the patient outweigh any expected benefit. The patient may be administered an antibody described herein and/or specifically disclosed below (e.g., humanized 2A4) in order to treat, retard, halt or reverse the progression of peripheral neuropathy associated with AL amyloidosis in the patient. Some regimens to treat, retard, halt or reverse the progression of peripheral neuropathy may advantageously require less of the antibody (e.g., humanized 2A4) to be administered to the patient than would be required to produce improvements in cardiac and/or renal function (if any).

Patients amenable to treatment also include those patients who have received, are currently receiving, or will later receive an alternate therapy for treatment of amyloid disease or an associated condition, such as, inflammatory diseases, chronic microbial infections, malignant neoplasms, inherited inflammatory diseases, and lymphoproliferative disorders. For example, patients may also receive or have received one or more of the therapeutic agents identified herein with respect to combination therapies. As an example, patients suffering from AL amyloidosis may also receive or have received bortezomib, melphalan, lenalidomide, dexamethasone, cyclophosphamide, pomalidomide, carfilzomib, doxorubicin, autologous transplant or combinations thereof. For those patients who have previously received alternate therapies for the treatment of amyloid disease, such therapies may or may not have been successful by the relevant clinical measures, and likely did not improve neuropathy. Additional examples of such therapies include (1) CyBorD, which is a combination therapy comprising cyclophosphamide, bortezomib and dexamethasone, (2) BMDex, which is a combination of bortezomib, melphalan and dexamethasone, (3) MDex, which is a combination of melphalan and dexamethasone, (4) LDex, which is a combination of lenalidomide and dexamethasone, (5) CLD, which is a combination of cyclophosphamide, lenalidomide and dexamethasone, and (6) PomDex, which is a combination of pomalidomide and dexamethasone. Some patients may be selected for treatment with the formulations herein only if previously treated with an alternative therapy.

Suitable antibodies, formulations and treatment regimes for the methods and uses disclosed herein are discussed in greater detail below.

### III. Pharmaceutical Formulations and Products

Provided herein are pharmaceutical formulations comprising a chimeric or humanized version of antibody 2A4 (ATCC Accession Number PTA-9662) or of antibody 7D8 (ATCC Accession Number PTA-9468) that specifically competes for binding to antigen *(i.e.,* human AA or AL protein) with 2A4 or 7D8, respectively, and/or that specifically binds to the same epitope as 2A4 or 7D8, and/or that specifically binds to an epitope comprising AEDS (SEQ ID NO: 18). Also provided are pharmaceutical formulations comprising murine antibody 2A4 or murine antibody 7D8, or antigen-binding fragments thereof. The antibody is present at a concentration within the range from about 1 mg/mL to about 100 mg/mL. The formulation is characterized by a pH within the range from about 6 to about 7 and comprises a histidine buffer at a concentration within the range from about 20 mM to about 30 mM, trehalose at a concentration within the range from about 210 mM to about 250 mM; and polysorbate 20 at a concentration within the range from about 0.005% to about 0.05% by weight. An exemplary antibody for use in the methods disclosed herein comprises three CDRs of a light chain variable region and/or three CDRs of a heavy chain variable region of antibody 2A4 or 7D8. For example, the antibody comprises a light chain variable region and/or a heavy chain variable region of antibody 2A4 or 7D8 or a chimeric or humanized version of antibody 2A4 or 7D8.

Humanized 2A4 is an IgG1, kappa isotype version of murine 2A4. In the course of specificity characterization of humanized 2A4, the antibody was found to also react with high affinity and in a conformation-dependent manner with light chain in light chain amyloid fibrils, but not with free light chain in circulation. Thus 2A4 antibodies specifically bind to pathologic amyloid forms of AL and SAA but do not bind to the parent molecules from which these pathologic forms are derived (*e.g*., SAA, native immunoglobulin light chain [LC], intact immunoglobulin [Ig]).

In some methods disclosed herein, the antibody can be administered as a pharmaceutical formulation, for example, comprising in addition to the antibody, a histidine buffer, trehalose, and polysorbate 20. In some such formulations used in the methods described above, the antibody is present at a concentration within the range from about 1 mg/mL to about 100 mg/mL; the histidine buffer is present at a concentration within the range from about 20 mM to about 30 mM; the trehalose is present at a concentration within the range from about 210 mM to about 250 mM; the polysorbate 20 present at a concentration within the range from about 0.005% to about 0.05% by weight; and the pH is within the range from about 6 to about 7. Some suitable formulations for the methods disclosed herein are described in greater detail below.

In some formulations, the antibody comprises a light chain variable region comprising an amino acid sequence set forth as any one of SEQ ID NOs: 1, 2, or 4. In some formulations, the antibody comprises a heavy chain variable region comprising an amino acid sequence set forth as SEQ ID NO: 3 or 5. In some formulations, the antibody comprises a light chain variable region comprising an amino acid sequence set forth as any one of SEQ ID NOs: 1, 2, or 4 and a heavy chain variable region comprising an amino acid sequence set forth as SEQ ID NO: 3 or 5. In some formulations, the antibody comprises a light chain variable region comprising an amino acid sequence set forth as SEQ ID NO: 1 and a heavy chain variable region comprising an amino acid sequence set forth as SEQ ID NO: 3. In some formulations, the antibody comprises a light chain variable region comprising an amino acid sequence set forth as SEQ ID NO: 4 and a heavy chain variable region comprising an amino acid sequence set forth as SEQ ID NO: 5. In some formulations, the antibody comprises a light chain variable region comprising an amino acid sequence set forth as SEQ ID NO: 2 and a heavy chain variable region comprising an amino acid sequence set forth as SEQ ID NO: 3.

In some formulations, the antibody comprises a light chain variable region comprising three complementarity determining regions set forth as SEQ ID NOs: 6, 7, and 8, and a heavy chain variable region comprising three complementarity regions set forth as SEQ ID NOs: 9, 10, and 11. In other formulations, the antibody comprises a light chain variable region comprising three complementarity determining regions set forth as SEQ ID NOs: 12, 7, and 8, and a heavy chain variable region comprising three complementarity regions set forth as SEQ ID NOs: 9, 10, and 11.

In other formulations, the antibody comprises light chain and heavy chain variable regions of a murine, chimeric, or humanized 2A4 antibody, or of a murine, chimeric, or humanized 7D8 antibody, as described in U.S. Patent No. 7,928,203 and PCT International Publication No. WO 2009/086539, and the light chain and heavy chain variable region sequences described in the referenced patent and publication are specifically incorporated by reference herein. Some formulations for the methods disclosed herein are described in U.S. Patent No. 9,089,529 and PCT International Publication No. WO 2013/063284.

In some formulations, the antibody comprises a light chain comprising an amino acid sequence set forth as SEQ ID NO: 13 or 21 and a heavy chain comprising an amino acid sequence set forth as any one of SEQ ID NOs: 14-16 and 24. The antibody can include, or not include, the leader sequences of the above-noted light chain and heavy chain amino acid sequences.

In other formulations, the antibody is a fragment of a 2A4 or 7D8 antibody, including chimeric and humanized versions thereof, such as a Fab fragment, a Fab' fragment, a F(ab')₂ fragment, a Fv fragment or a ScFv fragment.

Some antibodies specifically bind to aggregated amyloid protein without specifically binding to monomeric amyloid protein *(e.g.,* at least a 10-fold and usually at least 100-fold lower specific binding affinity for monomeric forms of the amyloid protein).

In some formulations, the antibody is present at a concentration within the range from about 5 mg/mL to about 100 mg/mL. In some formulations, the antibody is present at a concentration within the range from about 5 mg/mL to about 15 mg/mL. In some formulations, the antibody is present at a concentration within the range from about 25 mg/mL to about 75 mg/mL. For example, the antibody may be present at a concentration of about 10 mg/mL, or present at a concentration of about 50 mg/mL. The antibody may be present in a sterile liquid dosage form of about 50 mg/vial to about 500 mg/vial, or greater. For example, the antibody may be present in a sterile liquid dosage form of about 100 mg/vial.

Antibodies used in the disclosed formulations can be coupled with a therapeutic moiety, such as a cytotoxic agent, a radiotherapeutic agent, an immunomodulator, a second antibody *(e.g.,* to form an antibody heteroconjugate), or any other biologically active agent that facilitates or enhances the activity of a chimeric or humanized 2A4 or a chimeric or humanized 7D8 antibody. Representative therapeutic moieties include agent known to be useful for treatment, management, or amelioration of amyloid disease or symptoms of amyloid disease.

Therapeutic moieties and/or detectable substances may be coupled or conjugated directly to a murine, chimeric or humanized 2A4 antibody or a murine, chimeric or humanized 7D8 antibody, or indirectly, through an intermediate (*e.g*., a linker) using techniques known in the art. *See e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies 84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., Immunol. Rev., 1982, 62:119-58.

Antibodies used in the disclosed formulations also include modified forms of murine, chimeric or humanized 2A4 antibodies, or murine, chimeric or humanized 7D8 antibodies, which have increased *in vivo* half-lives relative to the corresponding unmodified antibodies. Such modified forms may be prepared, for example, by glycosylation, acetylation, pegylation, phosphorylation, amidation, derivatization by known protecting/blocking groups, proteolytic cleavage, linkage to a cellular ligand or other protein, *etc.* As one example, representative methods for antibody half-life extension are described in PCT International Publication No. WO 02/060919.

The histidine buffer may be present in some formulations at a concentration of about 25 mM. In some formulations, the histidine buffer comprises L-histidine and L-histidine HCl monohydrate. For example, in some formulations, L-histidine is present at a concentration within the range from about 16 mM to about 22 mM and L-histidine HCl monohydrate is present at a concentration within the range from about 4 mM to about 8 mM.

In some formulations, trehalose is present at a concentration from about 210 mM to about 250 mM, for example, about 230 mM. In some formulations, a different non-reducing sugar is used, such as sucrose, mannitol, or sorbitol.

In some formulations, polysorbate 20 is present at a concentration within the range of about from about 0.005% to about 0.05% by weight, for example, 0.005%, 0.01%, 0.015%, 0.02%, 0.025%, 0.03%, 0.035%, 0.04%, 0.045%, or 0.05%. Alternatively, in some formulations, polysorbate 20 is present at a concentration within the range of about from about 0.05 g/L, 0.1 g/L, 0.15 g/L, 0.2 g/L, 0.25 g/L, 0.3 g/L, 0.35 g/L, 0.4 g/L, 0.45 g/L, or 0.5 g/L. Some formulations include polysorbate 20 at a concentration of 0.2 g/L.

Some formulations are characterized by a pH within the range of about 6-7, for example, a pH of 6.0, 6.1, 6.2, 6.3, 6.4, 6.5, 6.6, 6.7, 6.8, 6.9, or 7.0. Some formulations have a pH of about 6.5. Some formulations are characterized by an osmolality of about 300 mOsm/kg. A bulking agent may also be included some formulations.

Typically, the formulations are sterile, for example, as accomplished by sterile filtration using a 0.2 µm or a 0.22 µm filter. The formulations disclosed herein are also generally stable upon freezing and thawing.

Optionally, formulations disclosed herein may further comprise other excipients, such as saccharides, polyols, and amino acids (*e.g*., arginine, lysine, and methionine). The present invention also provides formulations substantially free of surfactant, inorganic salts, additional sugars, and/or other excipients, *i.e.,* less than about less than 0.0005%, less than 0.0003%, or less than 0.0001% of such compounds.

An exemplary formulation comprises an antibody comprising a light chain comprising an amino acid sequence set forth as SEQ ID NO: 13 and a heavy chain comprising an amino acid sequence set forth as any one of SEQ ID NOs: 14, 15, or 16, which is present at a concentration of about 50 mg/mL, a histidine buffer present at a concentration of about 25 mM, trehalose present at a concentration of about 230 mM, polysorbate 20 present at a concentration of about 0.2 g/L, and a pH of about 6.5.

The methods disclosed herein involve pharmaceutical products comprising lyophilized antibody drug substance and instructions for reconstitution and use. For example, a representative pharmaceutical product can comprise: (a) a vial comprising about 100 mg antibody in powder form; (b) instructions for reconstitution of the antibody; and (c) instructions for preparing the reconstituted antibody for infusion, wherein (i) the antibody comprises a light chain comprising an amino acid sequence set forth as SEQ ID NO: 13 and a heavy chain comprising an amino acid sequence set forth as any one of SEQ ID NOs: 14-16; and (ii) the reconstitution instructions require reconstitution with water for injection to an extractable volume of 10 mL.

### IV. Treatment Regimes

As used herein, the terms "treat" and "treatment" refer to the alleviation or amelioration of one or more symptoms or effects associated with the disease, prevention, inhibition or delay of the onset of one or more symptoms or effects of the disease, lessening of the severity or frequency of one or more symptoms or effects of the disease, and/or increasing or trending toward desired outcomes as described herein.

Desired outcomes of the treatments disclosed herein vary according to the amyloid disease and patient profile and are readily determinable to those skilled in the art. Generally, desired outcomes include measurable indices such as reduction or clearance of pathologic amyloid fibrils, decreased or inhibited amyloid aggregation and/or deposition of amyloid fibrils, and increased immune response to pathologic and/or aggregated amyloid fibrils. Desired outcomes also include amelioration of amyloid disease-specific symptoms. For example, desired outcomes for the treatment of AL amyloidosis include a decrease in the incidence or severity of known symptoms, including organ dysfunction, peripheral and autonomic neuropathy, carpal tunnel syndrome, macroglossia, restrictive cardiomyopathy, arthropathy of large joints, immune dyscrasias, myelomas, as well as occult dyscrasias. Desired outcomes of the disclosed therapies are generally quantifiable measures as compared to a control or baseline measurement. As used herein, relative terms such as "improve," "increase," or "reduce" indicate values relative to a control, such as a measurement in the same individual prior to initiation of treatment described herein, or a measurement in a control individual or group. A control individual is an individual afflicted with the same amyloid disease as the individual being treated, who is about the same age as the individual being treated (to ensure that the stages of the disease in the treated individual and the control individual are comparable), but who has not received treatment using the disclosed antibody formulations. In this case, efficacy of the disclosed antibody formulations is assessed by a shift or trend away from measurable indices in the untreated control. Alternatively, a control individual is a healthy individual, who is about the same age as the individual being treated. In this case, efficacy of the disclosed antibody formulations is assessed by a shift or trend toward from measurable indices in the healthy control. Changes or improvements in response to therapy are generally statistically significant and described by a p-value less than or equal to 0.1, less than 0.05, less than 0.01, less than 0.005, or less than 0.001 may be regarded as significant.

In both asymptomatic and symptomatic patients, treatment according to the disclosed methods can begin at any time before or after the diagnosis of the underlying AL amyloid diseases. Treatment typically entails multiple dosages over a period of time. Treatment can be monitored by assaying antibody, or employing radiolabeled SAP Scintigraphy over time. If the response falls, a booster dosage may be indicated. The response of patients with AL amyloidosis to treatment can be monitored by assessing cardiac markers, such as NT-proBNP and/or troponin, serum creatine, and/or alkaline phosphatase; by performing serum free light chain (SFLC) assays, quantitative immunoglobulin assays, biopsies, serum protein electrophoresis (SPEP), urine protein electrophoresis (UPEP), serum, urine immunofixation electrophoresis (IFE), and/or organ imaging techniques. An exemplary complete response (CR) can be determined from response criteria including negative IFE of serum and urine, normal κ/λ ration and/or <5 % plasma cells in bone marrow. An exemplary very good partial response (VGPR) can be determined from a dFLC of < 40 mg/L. An exemplary partial response (PR) can be determined from a dFLC decrease of ≥ 50%. In the kidney, a response to treatment can be determined, for example, from a ≥ 50% reduction (e.g., > 0.5g/24 hours) in 24 hour urine protein excretion in the absence of either a reduction in eGFR of ≥ 25% or an increase in serum creatine of ≥ 0.5 mg/dL. In the liver, a response to treatment can be determined, for example, from a ≥ 50% reduction in initially elevated alkaline phosphatase or a ≥ 2 cm reduction in liver size on CT scan or MRI. In the heart, a response to treatment can be determined, for example, from a >30% and > 300 ng/L reduction in NT-proBNP in patients with baseline of NT-proBNP of > 650 ng/L. In the kidney, a response to treatment can be determined, for example, from a > 30% decrease in proteinuria or a decrease in proteinuria to < 0.5 g/24 hours in the absence of renal progression. Neuropathy responders are generally characterized by < 2 point increase in NIS-LL from baseline. Improvement in neuropathy (e.g., improved nerve function) is determined from a decrease in the NIS-LL from baseline.

Alleviation or amelioration of one or more symptoms or effects associated with an amyloidosis may be treated independently of one another. The term "independently" means that the antibody or antibody formulation can be administered in a dosage that is sufficient to treat one or more symptoms or effects (e.g., peripheral neuropathy) without treating all symptoms or effects or particular symptoms or effects (e.g., cardiac function, renal function).

The antibody formulation can be administered intravenously in dosage ranges from about 10 mg to about 5000 mg for the patient in question, such as, for example, about 10 mg, about 30 mg, about 100 mg, about 300 mg, about 1000 mg, about 2000 mg, or about 2500 mg. The antibody formulation can also be administered intravenously in dosage ranges from about 0.1 mg/kg to about 50 mg/kg, or from about 0.5 mg/kg to about 30 mg/kg, of the host body weight. For example, dosages can be about 0.5 mg/kg body weight, about 1.0 mg/kg, about 1.5 mg/kg, about 2.0 mg/kg, about 4.0 mg/kg, about 5.0 mg/kg, about 8.0 mg/kg, about 10 mg/kg, about 15 mg/kg, about 16 mg/kg, about 20 mg/kg, about 24 mg/kg, about 25 mg/kg, or about 30 mg/kg body weight. escalation for an individual patient can occur at the discretion of the prescriber in the absence of any clinically significant occurrence that the prescriber might reasonably believe would present an undue safety risk for the patient, such as, for example, Grade ≥ 3 nonhematologic toxicity, Grade ≥ 3 nausea, vomiting or diarrhea uncontrolled by maximum antiemetic/anti-diarrhea therapy, Grade 4 neutropenia lasting > 7 days in the absence of growth factor support, Grade 3 or 4 neutropenia of any duration accompanied with fever ≥ 38.5°C and/or systemic infection, or other Grade ≥ 4 hematologic toxicity.

Antibody is usually administered on multiple occasions. An exemplary treatment regime entails administration once per every two weeks, once a month, or once every 3 to 6 months. For example, patients can receive the antibody formulation once every four weeks as a cycle, for example every twenty-eight days. The dosing frequency can be adjusted depending on the pharmacokinetic profile of the antibody formulation in the patient. For example, the half-life of the antibody may warrant a two week frequency of dosing. In some methods, two or more monoclonal antibodies with different binding specificities are administered simultaneously, in which case the dosage of each antibody administered falls within the ranges indicated. Intervals between single dosages can be weekly, monthly or yearly. Intervals can also be irregular as indicated by measuring blood levels of antibody to amyloid protein *(e.g.,* AA) in the patient. In some methods, dosage is adjusted to achieve a plasma antibody concentration of about 1-1000 µg/mL or about 25-300 µg/mL. Alternatively, antibody can be administered as a sustained release formulation, in which case less frequent administration is required.

Dosage and frequency vary depending on the half-life of the antibody in the patient. In general, human antibodies show the longest half life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, until a partial or complete response is achieved, and/or until the patient shows lessening or amelioration of symptoms of disease. Thereafter, the patent can be administered a prophylactic regime.

The formulations disclosed herein may be provided in a dosage form that is suitable for parenteral (e.g., intravenous, intramuscular, subcutaneous) administration. As appropriate for particular applications, the formulation may be alternately provided in a dosage suitable for rectal, transdermal, nasal, vaginal, inhalant, ocular or other administration. The pharmaceutical formulations are typically prepared according to conventional pharmaceutical practice. *See e.g.,* Remington: The Science and Practice of Pharmacy, (19th ed.) ed. A. R. Gennaro, 1995, Mack Publishing Company, Easton, Pa. and Encyclopedia of Pharmaceutical Technology, eds. J. Swarbrick and J. C. Boylan, 1988-1999, Marcel Dekker, N.Y.

In some methods, the pharmaceutical formulation is administered intravenously or subcutaneously to the patient at a frequency of from about weekly to about quarterly, with a dosage of the antibody in the range of about 0.5 mg/kg to about 30 mg/kg. For example, the pharmaceutical formulation is administered to the patient intravenously every 28 days with an antibody dosage of about 24 mg/kg.

In some methods disclosed herein, the antibody is administered to the patient for at least 9 months, at least 12 months, or for a longer period of time. For example, the pharmaceutical formulation is administered to the patient for a duration effective to achieve or maintain less than a 2-point increase in NIS-LL from baseline. In some methods, the pharmaceutical formulation is administered to the patient for a duration effective to achieve or maintain at least a 10%, at least a 23%, at least a 35%, at least a 50%, or at least a 75% decrease in NIS-LL from baseline. In some methods disclosed herein, the pharmaceutical formulation is administered to the patient for a duration effective to achieve or maintain at least a 30% and 300 pg/mL decrease in NT-proBNP, which may be shorter or longer than the duration effective to achieve the NIS-LL changes described above.

In the methods provided herein, the treatment is continued for a period of time effective to achieve or maintain at least a 2 point increase in NIS-LL from baseline, for example, 9 or 12 months. In some methods, the treatment is continued for a period of time effective to achieve or maintain at least a 10% decrease in NIS-LL from baseline, for example, a 23%, 35%, 50% or 75% decrease in NIS-LL from baseline. In some methods, the duration of treatment is effective to achieve or maintain at least a 30% and 300 pg/mL decrease in NT-proBNP. In some methods, the intravenous administration is discontinued following achievement of a decrease in NIS-LL from baseline, for example, a 23%, 35%, 50% or 75% decrease. Some such patients may thereafter receive subcutaneous administration of the antibody in a regime effective to maintain the desired NIS-LL levels. In some methods described above, the patient previously received treatment with CRD, PomDex, CyBorD, BMDex, MDex, LDex, CLD or bortezomib.

Also disclosed herein are combination therapies for treatment or prophylaxis of amyloid disease, particularly AA amyloidosis and AL amyloidosis. Such combination therapies are performed by administering an antibody formulation disclosed herein in conjunction with one or more second therapeutic agents, such as another therapy to treat or effect prophylaxis of AA amyloidosis or AL amyloidosis, as the case may be. Combination therapies as disclosed herein may also be performed in conjunction with a second therapy is used to treat or effect prophylaxis of a disease or condition associated with amyloid disease, such as an inflammatory disease, a chronic microbial infection, a neoplasm (including malignant neoplasms), an inherited inflammatory disease, and/or a lymphoproliferative disorder. Numerous treatments are available in commercial use, in clinical evaluation, and in pre-clinical development, any of which could be selected for use in combination with the disclosed antibody formulations. Such treatments can be one or more compounds or treatments selected from, but not limited to several major categories, namely, (i) non-steroidal anti-inflammatory drugs (NSAIDs; *e.g*., detoprofen, diclofenac, diflunisal, etodolac, fenoprofen, flurbiprofen, ibuprofen, indomethacin, ketoprofen, meclofenameate, mefenamic acid, meloxicam, nabumeone, naproxen sodium, oxaprozin, piroxicam, sulindac, tolmetin, celecoxib, rofecoxib, aspirin, choline salicylate, salsalte, and sodium and magnesium salicylate); (ii) steroids (*e.g*., cortisone, dexamethasone, hydrocortisone, methylprednisolone, prednisolone, prednisone, triamcinolone); (iii) DMARDs, *i.e.,* disease modifying antirheumatic drugs (*e.g*., cyclosporine, azathioprine, methotrexate, leflunomide, cyclophosphamide, hydroxychloroquine, sulfasalazine, D-penicillamine, minocycline, and gold); (iv) recombinant proteins (*e.g*., ENBREL^{®} (etanercept, a soluble TNF receptor) and REMICADE^{®} (infliximab) a chimeric monoclonal anti-TNF antibody); (v) stem cell transplantation; and/or (vi) chemotherapy. Patients with AL amyloidosis may also receive treatment regimes that include drugs or combinations of drugs often used to treat hematological malignancies, such as melphalan, prednisone, dexamethasone, lenalidomide (REVL1M1D^{®}) and proteosome inhibitors such as bortezomib (VELCADE^{®}), and carfilzomib (KYPROLIS^{®}), at dosages in the range of the standard of care.

The duration of the combination therapy depends on the type of amyloid disease being treated, any underlying disease associated with the amyloid disease, the age and condition of the patient, the stage and type of the patient's disease, how the patient responds to the treatment, *etc.* A medical doctor can observe the therapy's effects closely and make any adjustments as needed.

When performing a combination therapy, the two or more drug substances are administered simultaneously or sequentially in any order, *i.e.,* a formulation disclosed herein is administered prior to administering a second drug substance, concurrently with a second drug substance, or subsequent to administration of a second drug substance. For example, a combination therapy may be performed by administering a first therapy prior to *(e.g.,* 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to *(e.g.,* 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) administering a second agent/therapy.

The dosage, frequency and mode of administration of each component of the combination can be controlled independently. For example, one therapeutic agent/therapy may be administered orally three times per day, while the second therapeutic agent/therapy may be administered intramuscularly once per day. Combination therapy may be given in on-and-off cycles that include rest periods. The compounds may also be admixed or otherwise formulated together such that one administration delivers both compounds. In this case, each therapeutic agent is generally present in an amount of 1-95% by weight of the total weight of the composition. Alternatively, an antibody formulation disclosed herein and a second therapeutic agent can be formulated separately and in individual dosage amounts. Drug combinations for treatment can be provided as components of a pharmaceutical pack.

Preferably, the disclosed combination therapies elicit a synergistic therapeutic effect, i.e., an effect greater than the sum of their individual effects or therapeutic outcomes. Measurable therapeutic outcomes are described herein. For example, a synergistic therapeutic effect may be an effect of at least about two-fold greater than sum of the therapeutic effects elicited by the single agents of a given combination, or at least about five-fold greater, or at least about ten-fold greater, or at least about twenty-fold greater, or at least about fifty-fold greater, or at least about one hundred-fold greater. A synergistic therapeutic effect may also be observed as an increase in therapeutic effect of at least 10% compared to the sum of the therapeutic effects elicited by the single agents of a given combination, or at least 20%, or at least 30%, or at least 40%, or at least 50%, or at least 60%, or at least 70%, or at least 80%, or at least 90%, or at least 100%, or more. A synergistic effect is also an effect that permits reduced dosing of therapeutic agents when they are used in combination.

### EXAMPLES

The following examples have been included to illustrate modes disclosed herein. Certain aspects of the following examples are described in terms of techniques and procedures found or contemplated by the present co-inventors to work well in the practice disclosed herein. In light of the present disclosure and the general level of skill in the art, those of skill appreciate that the following examples are intended to be exemplary only and that numerous changes, modifications, and alterations may be employed without departing from the scope of the disclosure.

### Example 1. Clinical Assessment of Humanized 2A4 (NEOD001)

A Phase 1/2 clinical trial was designed to determine a maximum tolerated dose (MTD) and/or the Phase 2 recommended dose (P2RD) of humanized 2A4 (NEOD001) in subjects with AL amyloidosis. Dosing began at 0.5 mg/kg and escalated to a high of 24 mg/kg with a maximum dose of 2500 mg. Initially, NEOD001 was given intravenously as a single agent every 28 days until progression of organ function or unacceptable treatment related toxicity or withdraw of consent.

In the dose-escalation phase of the study following a standard 3 + 3 design, 27 patients with AL amyloidosis were treated with NEOD001. The seven cohorts of the dose-escalation phase were 0.5 mg/kg, 1 mg/kg, 2 mg/kg, 4 mg/kg, 8 mg/kg, 16 mg/kg and 24 mg/kg (or maximum dose of 2500mg). An additional 42 patients with AL amyloidosis and prospectively defined organ involvement were included in expansion cohorts, for a total of 69 patients in the study. The cardiac cohort included 15 patients and cardiac involvement was defined by an elevated NT-proBNP of ≥650 pg/mL in the absence of renal failure. Patients with an NT-proBNP level >7,000 pg/mL were excluded. The exploratory endpoint was NT-proBNP best response. The renal cohort included 16 patients and renal involvement was defined by proteinuria >0.5 g/day in a 24 hour urine collection. The exploratory endpoint was proteinuria best response. The peripheral neuropathy cohort included 11 patients with a positive sural nerve biopsy or evidence of a typical sensorimotor peripheral neuropathy due to AL amyloidosis. The exploratory endpoint was the neuropathy impairment score-lower limbs (NIS-LL), baseline to Month 10. The NIS-LL scores various attributes of peripheral nervous system function, including sensation, muscle power and tendon reflexes (Bril, Eur. Neorol., 1999; 41 Suppl 1:8-13, Coelho et al, Neurology, 2012; 799(8):785-92).

Table 1 sets forth the patient characteristics.

**Table 1**

| Patient Characteristics | |
|---|---|
| | **All Patients (N = 69)** |
| Median age, years (range) | 60 (38-81) |
| Gender (% male) | 42 (61%) |
| Median time since initial diagnosis, years (range) | 2.8 (0.0-16.0) |
| Median no. previous regimens (range) | 2.0 (0-7) |
| No. organ systems involved, n (%) | |
| 1 | 22 (32) |
| 2 | 29(42) |
| ≥3 | 18(26) |
| Median months since last PCD treatment, (range) | 6.5 (0.6-85.8) |
| Median NT-proBNP (pg/mL) at screening, (range) | |
| Cardiac evaluable escalation phase (n=14) | 1103 (651-3588) |
| Cardiac expansion cohort (n=15) | 1920 (766-5620) |
| Total cardiac evaluable (n=36) | 1507 (651-5620) |

The drug product was safe and well tolerated with no dose-limiting toxicities, no detection of anti-drug antibodies and no treatment-related serious adverse events.

### Example 2. Cardiac Response to NEOD001

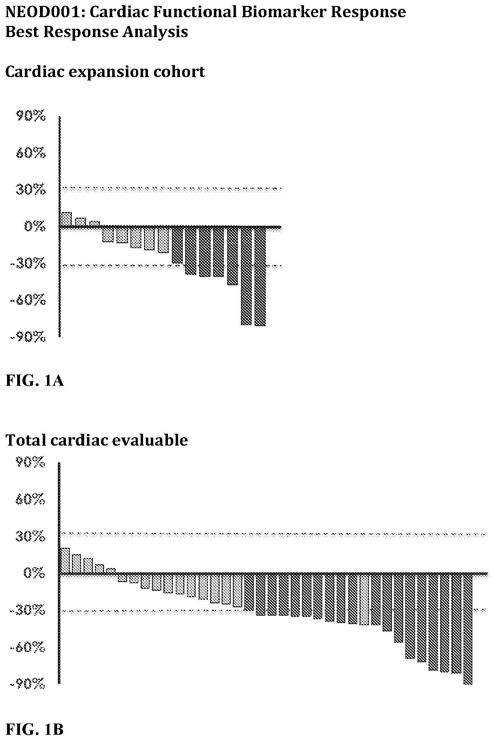

Among the cardiac expansion cohort (N=15), there were 7 responders (47%) and 8 stable patients (53%) as shown in FIG. 1A. The asterisk indicates a 30% decline, 453 pg/mL reduction from baseline for one of the responders. Cardiac evaluable patients include patients within the cardiac cohort, which was a prospectively defined cohort of cardiac patients, as well as patients from other cohorts that could be evaluated for cardiac response. Among the total cardiac evaluable patients (N=36), there were 19 responders (53%) and 17 stable patients (47%) as shown in FIG. IB. Evaluable patients had baseline NT-proBNP ≥650 pg/mL without progressive renal dysfunction. Response was defined as >30% and >300 pg/mL decrease in NT-proBNP. Progression was defined as >30% and >300 pg/mL increase in NT-proBNP. Stable disease was defined as neither response nor progression. Comenzo et al, Leukemia (2012) 26, 2317-2325; Palladini et al., J. Clin. Oncology (2012) 30 (36), 4541-4549.

As shown in Table 2, the cardiac response rate of organ relapse/refractory patients treated with NEOD001 is 53%, compared to the 0-15% rate observed in such patients treated with cyclophosphamide (CRD) or pomalidomide/dexamethasone (PomDex) (Palladini et al., Haematologica 2013 and Dispenzieri et al., Blood, 2012) or or the 17-27% cardiac response rate of newly diagnosed patients treated with cyclophosphamide, bortezomib and dexamethasone (CyBorD), bortezomib, melphalan and dexamethasone (BMDex), melphalan and dexamethasone (MDex) or other treatments (Palladini et al, Blood (2015) 126(5), 612-615 and Comenzo et al).

**Table 2**

| Cardiac Response Rates in Organ Relapse/Refractory Patients Treated with NEOD001 | | |
|---|---|---|
| Treatment | Number of Patients | Cardiac Response |
| CRD | 21 | 0% |
| PomDex | 33 | 15% |
| NEOD001 | 36 | 53% |

### Example 3. Renal Response to NEOD001

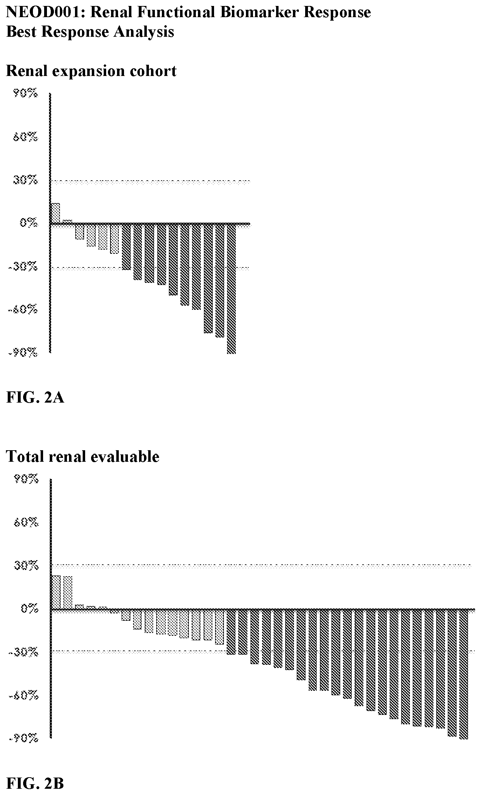

Among the renal expansion cohort (N=16), there were 10 responders (63%) and 6 stable patients (37%) as shown in FIG. 2A. Renal evaluable patients include patients within the renal cohort, which was a prospectively defined cohort of renal patients, as well as patients from other cohorts that could be evaluated for renal response. Among the total renal evaluable patients, there were 22 responders (63%) and 13 stable patients (37%) as shown in FIG. 2B. Evaluable patients had baseline proteinuria ≥0.5 g/24 hours. Response was defined as >30% decrease in proteinuria or a decrease in proteinuria to <0.5 g/24 hours in the absence of renal progression. Progression was defined as >25% worsening in eGFR. Stable disease was defined as neither response nor progression. Palladini et al.

As shown in Table 3, the renal response rate of organ relapse/refractory patients treated with NEOD001 is greater than 63%, compared to the 17-29% rate observed in such patients treated with lenalidomide and dexamethasone (LDex), cyclophosphamide, lenalidomide and dexamethasone (CLD), PomDex or bortezomib (Bor) (Mahmood et al, Br. J. Haematology, 2014; 166:842-848, Palladini et al., Haematologica, 2013;98:433-436, Dispenzieri et al, Blood, 2012;119:4397-4404, Reece et al., Blood, 2011; 118:865-873) or the 21-29% response rate of newly diagnosed patients treated with CyBorD, BMDex or MDex (Palladini et al., Blood, 2015;126(23) abstract 190, Palladini et al., Blood, 2015; 126:612-615).

### Example 4. Peripheral Neuropathy Response to NEOD001

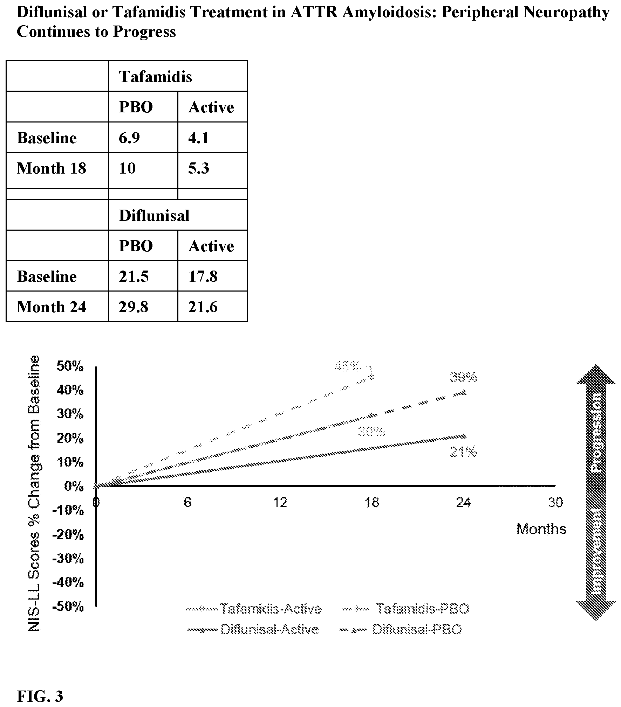

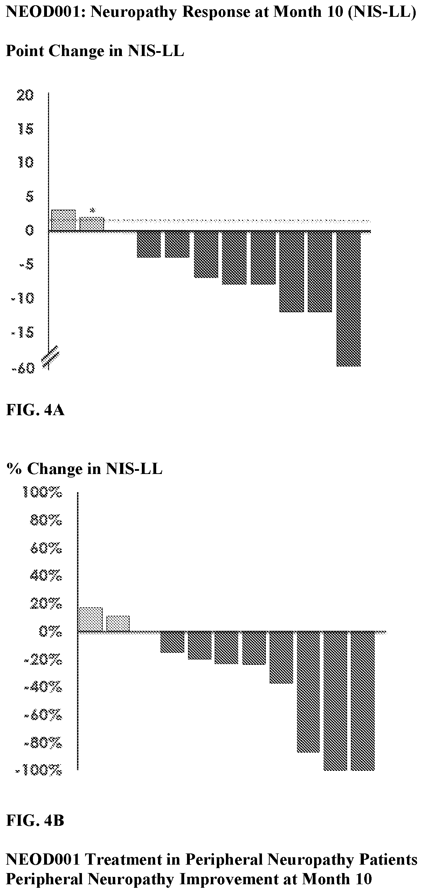

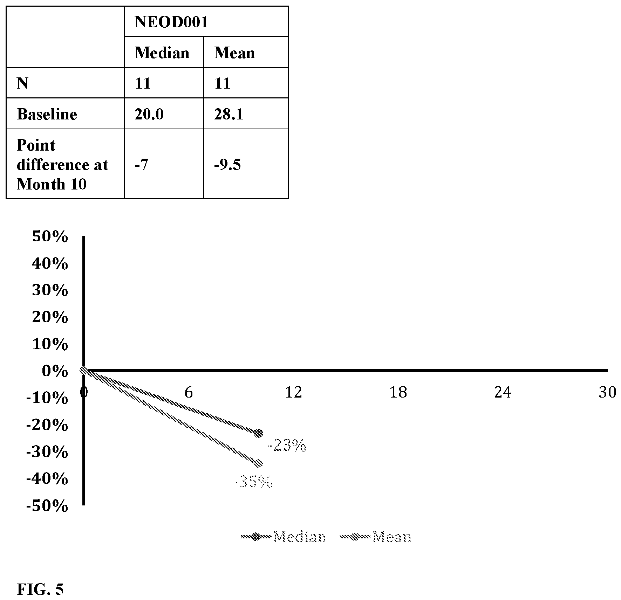

There have been no reported cases of improvement in peripheral neuropathy following treatment of AL amyloidosis or other amyloidosis such as ATTR amyloidosis. At best, such treatments may reduce the rate of progression. For example, as shown in FIG. 3, the rate of progression in ATTR patients treated with Tafamidis was lowered to 39% compared to 45% in untreated patients, and the rate of progression in ATTR patients treated with Diflunisal was lowered to 21% compared to 30% in untreated patients (Coelho et al., Neurology, 2012, Coelho et al, J. Neurol., 2013, Berk et al, JAMA, 2013). However, in stark contrast to the results observed with other treatments, the instant inventors surprisingly discovered that NEOD001 treatment actually improved peripheral neuropathy. As shown in FIG. 4A and FIG. 4B, among the neuropathy expansion cohort (N=11), there were 9 responders (82%) and 2 progressors (18%). One responder had no change from baseline. Neuropathy responders were defined as <2 point increase in NIS-LL from baseline. These response criteria were established in patients with diabetic neuropathy and are currently in use in clinical trials for diabetic neuropathy and TTR polyneuropathy. Thus, as indicated in FIG. 5, the mean peripheral neuropathy improvement at Month 10 (following 9 months of treatment) is a -35% and the median improvement is -23%. This is the first time improvement in peripheral neuropathy has been evidenced in patients with AL amyloidosis.

### Example 5. Patient Specific Cardiac, Renal and Neuropathy Response

Of the 11 patients in the peripheral neuropathy cohort, 3 were cardiac evaluable and one was cardiac and renal evaluable. Of the 3 cardiac evaluable patients, two patients had both cardiac and neuropathy improvement, one had neuropathy improvement but not cardiac improvement. The cardiac and renal evaluable patient had renal improvement, but not cardiac or neuropathy improvement. Thus, while NEOD001 can improve function across at least 3 organ systems in a population of AL amyloidosis patients, treatment effects can vary for the individual patient. NEOD001 can improve function in one or more organ systems independent of other organ systems, for example, restoring neural function in patients lacking a cardiac response.

While this invention has been disclosed with reference to specific embodiments, other embodiments and variations of this invention can be devised by others skilled in the art without departing from the scope of the invention. The appended claims include all such embodiments and equivalent variations.

## Claims

1. An antibody comprising a light chain variable region comprising three complementarity determining regions set forth as SEQ ID NOs: 6, 7 and 8, and a heavy chain variable region comprising three complementarity determining regions set forth as SEQ ID NOs: 9, 10 and 11 for use in a method:
(A) of treating peripheral neuropathy in a patient with peripheral neuropathy associated with AL amyloidosis; or
(B) of independently treating peripheral neuropathy in a patient with AL amyloidosis, wherein:
(a) the patient presents with peripheral neuropathy;
(b) the patient has not shown any cardiac response to an effective dosage of the antibody when previously administered;
(c) the patient has not shown any renal response to an effective dosage of the antibody when previously administered;
(d) the patient previously received treatment with a different agent that did not affect the patient's peripheral neuropathy; and/or
(e) the patient is receiving treatment with a different agent that does not affect the patient's peripheral neuropathy;
and further wherein SEQ ID NOs: 6, 7, 8, 9, 10 and 11 have the following sequences (using single letter amino acid codes): RSSQSLVHSTGNTYLH, KVSNRFS, SQSTHVPFT, GFTFNTYAMY, RIRSKSNNYAIYYADSVKD and PYSDSFAY, respectively.

2. The antibody for use of claim 1(A), wherein:
(i) progression of the peripheral neuropathy is reversed; or
(ii) the duration of treatment is effective to achieve or maintain less than a 2-point increase in NIS-LL from baseline.

3. The antibody for use of claim 1(A), wherein the antibody is humanized.

4. The antibody for use of claim 1, wherein the light chain variable region comprises the amino acid sequence set forth as SEQ ID NO: 4 and the heavy chain variable region comprises the amino acid sequence set forth as SEQ ID NO: 5; and further wherein SEQ ID NOs: 4 and 5 have the following sequences (using single letter amino acid codes):
DVVMTQSPLSLPVTPGEPASISCRSSQSLVHSTGNTYLHWYLQKPGQSP QLLIYKVSNRFSGVPDRFSGSGSGTDFTLKISRVEAEDVGVYYCSQSTHVPFTFG GGTKVEIK and
EVQLVESGGGLVQPGGSLRLSCAASGFTFNTYAMYWIRQAPGKGLEW VARIRSKSNNYAIYYADSVKDRFTISRDDSKNSLYLQMNSLKTEDTAVYYCARP YSDSFAYWGQGTLVTVSS, respectively.

5. The antibody for use of claim 4, wherein the patient previously received treatment with melphalan, prednisone, dexamethasone, bortezomib, cyclophosphamide, lenalidomide, doxorubicin, autologous transplant or a combination thereof.

6. The antibody for use of claim 1(B), wherein:
(i) the different agent is melphalan, prednisone, dexamethasone, bortezomib, cyclophosphamide, lenalidomide, or a combination thereof; or
(ii) the patient presented with a symptom other than peripheral neuropathy; and/or
(iii) the patient presented with peripheral neuropathy; and/or
(iv) the patient has not shown any cardiac or renal response to a previous administration of the antibody.

7. The antibody for use of claim 1, formulated as a pharmaceutical formulation comprising:
a) the antibody at a concentration within the range from about 1mg/mL to about 100 mg/mL;
b) histidine buffer at a concentration within the range from about 20 mM to about 30 mM;
c) trehalose at a concentration within the range from about 210 mM to about 250 mM; and
d) polysorbate 20 at a concentration within the range from about 0.005% to about 0.05% by weight; and
wherein the pharmaceutical formulation is **characterized by** a pH within the range from about 6 to about 7.

8. The antibody for use of claim 7, comprising a dosage from about 0.5 mg/kg to about 30 mg/kg and wherein the antibody is administered intravenously or subcutaneously at a frequency of from about weekly to about quarterly.

9. The antibody for use of claim 8, wherein:
a) the antibody is present at a concentration of about 50 mg/mL;
b) the histidine buffer is present at a concentration of about 25 mM;
c) the trehalose is present at a concentration of about 230 mM;
d) the polysorbate 20 is present at a concentration of about 0.2 g/L; and
wherein the pH is about 6.5.

10. The antibody for use of claim 8, wherein the dosage is about 24 mg/kg and the antibody is administered intravenously every 28 days.

11. The antibody for use of claim 10, wherein:
(i) the duration of the treatment is at least 9 months; or
(ii) the duration of the treatment is at least 12 months; or
(iii) the duration of treatment is effective to achieve or maintain less than a 2-point increase in NIS-LL from baseline; or
(iv) the duration is effective to achieve or maintain at least a 10% decrease in NIS-LL from baseline; or
(v) the duration is effective to achieve or maintain at least a 23% decrease in NIS-LL from baseline; or
(vi) the duration is effective to achieve or maintain at least a 35% decrease in NIS-LL from baseline; or
(vii) the duration is effective to achieve or maintain at least a 50% decrease in NIS-LL from baseline; or
(viii) the duration is effective to achieve or maintain at least a 75% decrease in NIS-LL from baseline; or
(ix) the duration is effective to achieve or maintain at least a 75% decrease in NIS-LL from baseline and the duration is effective to achieve or maintain at least a 30% and 300 pg/mL decrease in NT-proBNP.

12. The antibody for use of claim 5, 6(i) or 10, wherein the patient previously received treatment with CRD, PomDex, CyBorD, BMDex, MDex, LDex, CLD or bortezomib.

13. The antibody for use of claim 1, wherein the antibody is a Fab, Fab', F(ab')₂, F(ab)c, or Fv.

## Patentansprüche

1. Antikörper, umfassend eine variable Region der leichten Kette, umfassend drei komplementaritätsbestimmende Regionen, dargelegt als SEQ ID NO: 6, 7 und 8, und eine variable Region der schweren Kette, umfassend drei komplementaritätsbestimmende Regionen, dargelegt als SEQ ID NO: 9, 10 und 11, zur Verwendung in einem Verfahren:
(A) des Behandelns von peripherer Neuropathie in einem Patienten mit peripherer Neuropathie im Zusammenhang mit AL-Amyloidose; oder
(B) des unabhängigen Behandelns von peripherer Neuropathie in einem Patienten mit AL-Amyloidose, wobei:
(a) der Patient mit peripherer Neuropathie vorstellig wird;
(b) der Patient keine kardiale Antwort auf eine wirksame Dosis des Antikörpers gezeigt hat, wenn sie zuvor verabreicht wurde;
(c) der Patient keine renale Antwort auf eine wirksame Dosis des Antikörpers gezeigt hat, wenn sie zuvor verabreicht wurde;
(d) der Patient zuvor eine Behandlung mit einem anderen Mittel erhalten hat, das die periphere Neuropathie des Patienten nicht beeinflusst hat; und/oder
(e) der Patient eine Behandlung mit einem anderen Mittel erhält, das die periphere Neuropathie des Patienten nicht beeinflusst;
und wobei ferner SEQ ID NO: 6, 7, 8, 9, 10 und 11 die folgenden Sequenzen haben (unter Verwendung von Aminosäurecodes mit einzelnen Buchstaben): RSSQSLVHSTGNTYLH, KVSNRFS, SQSTHVPFT, GFTFNTYAMY, RIRSKSNNYAIYYADSVKD bzw. PYSDSFAY.

2. Antikörper zur Verwendung nach Anspruch 1(A), wobei:
(i) Progression der peripheren Neuropathie rückgängig gemacht wird; oder
(ii) die Dauer der Behandlung wirksam ist, um weniger als eine 2-Punkt-Zunahme in NIS-LL vom Ausgangswert zu erreichen oder aufrechtzuhalten.

3. Antikörper zur Verwendung nach Anspruch 1(A), wobei der Antikörper humanisiert ist.

4. Antikörper zur Verwendung nach Anspruch 1, wobei die variable Region der leichten Kette die Aminosäuresequenz umfasst, die als SEQ ID NO: 4 dargelegt ist, und die variable Region der schweren Kette die Aminosäuresequenz umfasst, die als SEQ ID NO: 5 dargelegt ist; und wobei ferner SEQ ID NO: 4 und 5 die folgenden Sequenzen haben (unter Verwendung von Aminosäurecodes mit einzelnen Buchstaben):
DVVMTQSPLSLPVTPGEPASISCRSSQSLVHSTGNTYLHWYLQKPGQSPQLLIY KVSNRFSGVPDRFSGSGSGTDFTLKISRVEAEDVGVYYCSQSTHVPFTFGGGTKVEIK bzw.
EVQLVESGGGLVQPGGSLRLSCAASGFTFNTYAMYWIRQAPGKGLEWVARIR SKSNNYAIYYADSVKDRFTISRDDSKNSLYLQMNSLKTEDTAVYYCARPYSDSFAYW GQGTLVTVSS.

5. Antikörper zur Verwendung nach Anspruch 4, wobei der Patient zuvor eine Behandlung mit Melphalan, Prednison, Dexamethason, Bortezomib, Cyclophosphamid, Lenalidomid, Doxorubicin, autologem Transplantat oder einer Kombination davon erhalten hat.

6. Antikörper zur Verwendung nach Anspruch 1(B), wobei:
(i) das andere Mittel Melphalan, Prednison, Dexamethason, Bortezomib, Cyclophosphamid, Lenalidomid oder eine Kombination davon ist; oder
(ii) der Patient mit einem Symptom vorstellig wird, das nicht periphere Neuropathie ist; und/oder
(iii) der Patient mit peripherer Neuropathie vorstellig wird; und/oder
(iv) der Patient keine kardiale oder renale Antwort auf eine vorherige Verabreichung des Antikörpers gezeigt hat.

7. Antikörper zur Verwendung nach Anspruch 1, formuliert als eine pharmazeutische Formulierung, umfassend:
a) den Antikörper in einer Konzentration in dem Bereich von etwa 1 mg/ml bis etwa 100 mg/ml;
b) Histidinpuffer in einer Konzentration in dem Bereich von etwa 20 mM bis etwa 30 mM;
c) Trehalose in einer Konzentration in dem Bereich von etwa 210 mM bis etwa 250 mM; und
d) Polysorbat 20 in einer Konzentration in dem Bereich von etwa 0,005 Gew.-% bis etwa 0,05 Gew.-%; und
wobei die pharmazeutische Formulierung durch einen pH-Wert in dem Bereich von etwa 6 bis etwa 7 gekennzeichnet ist.

8. Antikörper zur Verwendung nach Anspruch 7, umfassend eine Dosierung von etwa 0,5 mg/kg bis etwa 30 mg/kg, und wobei der Antikörper intravenös oder subkutan in einer Häufigkeit von etwa wöchentlich bis etwa vierteljährlich verabreicht wird.

9. Antikörper zur Verwendung nach Anspruch 8, wobei:
a) der Antikörper in einer Konzentration von etwa 50 mg/ml vorhanden ist;
b) der Histidinpuffer in einer Konzentration von etwa 25 mM vorhanden ist;
c) die Trehalose in einer Konzentration von etwa 230 mM vorhanden ist;
d) das Polysorbat 20 in einer Konzentration von etwa 0,2 g/l vorhanden ist; und wobei der pH-Wert etwa 6,5 ist.

10. Antikörper zur Verwendung nach Anspruch 8, wobei die Dosierung etwa 24 mg/kg beträgt und der Antikörper intravenös alle 28 Tage verabreicht wird.

11. Antikörper zur Verwendung nach Anspruch 10, wobei:
(i) die Dauer der Behandlung mindestens 9 Monate beträgt; oder
(ii) die Dauer der Behandlung mindestens 12 Monate beträgt; oder
(iii) die Dauer der Behandlung wirksam ist, um weniger als eine 2-Punkt-Zunahme in NIS-LL vom Ausgangswert zu erreichen oder aufrechtzuhalten; oder
(iv) die Dauer wirksam ist, um mindestens eine 10%-ige Abnahme in NIS-LL vom Ausgangswert zu erreichen oder aufrechtzuhalten; oder
(v) die Dauer wirksam ist, um mindestens eine 23%-ige Abnahme in NIS-LL vom Ausgangswert zu erreichen oder aufrechtzuhalten; oder
(vi) die Dauer wirksam ist, um mindestens eine 35%-ige Abnahme in NIS-LL vom Ausgangswert zu erreichen oder aufrechtzuhalten; oder
(vii) die Dauer wirksam ist, um mindestens eine 50%-ige Abnahme in NIS-LL vom Ausgangswert zu erreichen oder aufrechtzuhalten; oder
(viii) die Dauer wirksam ist, um mindestens eine 75%-ige Abnahme in NIS-LL vom Ausgangswert zu erreichen oder aufrechtzuhalten; oder
(ix) die Dauer wirksam ist, um mindestens eine 75%-ige Abnahme in NIS-LL vom Ausgangswert zu erreichen oder aufrechtzuhalten und die Dauer wirksam ist, um mindestens eine Abnahme um 30 % und 300 pg/ml in NT-proBNP zu erreichen oder aufrechtzuhalten.

12. Antikörper zur Verwendung nach Anspruch 5, 6(i) oder 10, wobei der Patient zuvor eine Behandlung mit CRD, PomDex, CyBorD, BMDex, MDex, LDex, CLD oder Bortezomib erhalten hat.

13. Antikörper zur Verwendung nach Anspruch 1, wobei der Antikörper Fab, Fab', F(ab')₂, F(ab)c oder Fv ist.

## Revendications

1. Anticorps comprenant une région variable de chaîne légère comprenant trois régions de détermination de complémentarité indiquées en SEQ ID N° : 6, 7 et 8, et une région variable de chaîne lourde comprenant trois régions de détermination de complémentarité indiquées en SEQ ID N° : 9, 10, et 11, destiné à être utilisé dans un procédé :
(A) de traitement de neuropathie périphérique chez un patient atteint de neuropathie périphérique associée à l'amylose AL ; ou
(B) de traitement indépendant de neuropathie périphérique chez un patient atteint d'amylose AL, dans lequel :
(a) le patient présente une neuropathie périphérique ;
(b) le patient n'a montré aucune réponse cardiaque à une administration précédente d'une posologie efficace de l'anticorps ;
(c) le patient n'a montré aucune réponse rénale à une administration précédente d'une posologie efficace de l'anticorps ;
(d) le patient a précédemment reçu un traitement avec un agent différent qui n'a pas affecté la neuropathie périphérique du patient ; et/ou
(e) le patient reçoit un traitement avec un agent différent qui n'affecte pas la neuropathie périphérique du patient ;
et en outre dans lequel lesdites SEQ ID N° : 6, 7, 8, 9, 10 et 11 présentent les séquences suivantes (à l'aide des codes d'acides aminés à une lettre) : RSSQSLVHSTGNTYLH, KVSNRFS, SQSTHVPFT, GFTFNTYAMY, RIRSKSNNYAIYYADSVKD et PYSDSFAY, respectivement.

2. Anticorps destiné à être utilisé selon la revendication 1(A), dans lequel :
(i) la progression de la neuropathie périphérique est inversée ; ou
(ii) la durée du traitement est efficace pour obtenir ou maintenir une augmentation inférieure à 2 points de NIS-LL par rapport à la valeur initiale.

3. Anticorps destiné à être utilisé selon la revendication 1(A), dans lequel l'anticorps est humanisé.

4. Anticorps destiné à être utilisé selon la revendication 1, dans lequel la région variable de chaîne légère comprend la séquence d'acides aminés indiquée en SEQ ID N° : 4, et la région variable de chaîne lourde comprend la séquence d'acides aminés indiquée en SEQ ID N° : 5 ; et en outre dans lequel SEQ ID N° : 4 et 5 présentent les séquences suivantes (à l'aide des codes d'acides aminés à une lettre) :
DVVMTQSPLSLPVTPGEPASISCRSSQSLVHSTGNTYLHWYLQKPGQ SPQLLIYKVSNRFSGVPDRFSGSGSGTDFTLKISRVEAEDVGVYYCSQSTHV PFTFGGGTKVEIK et
EVQLVESGGGLVQPGGSLRLSCAASGFTFNTYAMYWIRQAPGKGLE WVARIRSKSNNYAIYYADSVKDRFTISRDDSKNSLYLQMNSLKTEDTAVY YCARPYSDSFAYWGQGTLVTVSS, respectivement.

5. Anticorps destiné à être utilisé selon la revendication 4, dans lequel le patient a précédemment reçu un traitement avec du melphalan, de la prednisone, de la dexaméthasone, du bortézomib, du cyclophosphamide, du lénalidomide, de la doxorubicine, une greffe autologue ou une combinaison de ceux-ci.

6. Anticorps destiné à être utilisé selon la revendication 1(B), dans lequel :
(i) l'agent différent est le melphalan, la prednisone, la dexaméthasone, le bortézomib, le cyclophosphamide, le lénalidomide, ou une combinaison de ceux-ci ; ou
(ii) le patient présentait un symptôme autre qu'une neuropathie périphérique ; et/ou
(iii) le patient présentait une neuropathie périphérique ; et/ou
(iv) le patient n'a montré aucune réponse cardiaque ou rénale à une administration précédente de l'anticorps.

7. Anticorps destiné à être utilisé selon la revendication 1, formulé en tant que formulation pharmaceutique comprenant :
a) l'anticorps à une concentration dans la plage d'environ 1 mg/mL à environ 100 mg/mL ;
b) un tampon d'histidine à une concentration dans la plage d'environ 20 mM à environ 30 mM ;
c) du tréhalose à une concentration dans la plage d'environ 210 mM à environ 250 mM ; et
d) du polysorbate 20 à une concentration dans la plage d'environ 0,005 % à environ 0,05 % en poids ; et,
dans lequel la formulation pharmaceutique est **caractérisée par** un pH dans la plage d'environ 6 à environ 7.

8. Anticorps destiné à être utilisé selon la revendication 7, comprenant une posologie d'environ 0,5 mg/kg à environ 30 mg/kg et dans lequel l'anticorps est administré par voie intraveineuse ou sous-cutanée à une fréquence d'environ une fois par semaine à environ une fois par trimestre.

9. Anticorps destiné à être utilisé selon la revendication 8, dans lequel :
a) l'anticorps est présent à une concentration d'environ 50 mg/mL ;
b) le tampon d'histidine est présent à une concentration d'environ 25 mM;
c) le tréhalose est présent à une concentration d'environ 230 mM ;
d) le polysorbate 20 est présent à une concentration d'environ 0,2 g/L ; et,
dans lequel le pH est d'environ 6,5.

10. Anticorps destiné à être utilisé selon la revendication 8, dans lequel la posologie est d'environ 24 mg/kg et l'anticorps est administré par voie intraveineuse tous les 28 jours.

11. Anticorps destiné à être utilisé selon la revendication 10, dans lequel :
(i) la durée du traitement est d'au moins 9 mois ; ou
(ii) la durée du traitement est d'au moins 12 mois ; ou
(iii) la durée du traitement est efficace pour obtenir ou maintenir une augmentation inférieure à 2 points de NIS-LL par rapport à la valeur initiale ; ou
(iv) la durée est efficace pour obtenir ou maintenir au moins une diminution de 10 % de NIS-LL par rapport à la valeur initiale ; ou
(v) la durée est efficace pour obtenir ou maintenir au moins une diminution de 23 % de NIS-LL par rapport à la valeur initiale ; ou
(vi) la durée est efficace pour obtenir ou maintenir au moins une diminution de 35 % de NIS-LL par rapport à la valeur initiale ; ou
(vii) la durée est efficace pour obtenir ou maintenir au moins une diminution de 50 % de NIS-LL par rapport à la valeur initiale ; ou
(viii) la durée est efficace pour obtenir ou maintenir au moins une diminution de 75 % de NIS-LL par rapport à la valeur initiale ; ou
(ix) la durée est efficace pour obtenir ou maintenir au moins une diminution de 75 % de NIS-LL par rapport à la valeur initiale et la durée est efficace pour obtenir ou maintenir une diminution d'au moins 30 % et 300 pg/mL de NT-proBNP.

12. Anticorps destiné à être utilisé selon la revendication 5, 6(i) ou 10, dans lequel le patient a précédemment reçu un traitement avec CRD, PomDex, CyBorD, BMDex, MDex, LDex, CLD ou du bortézomib.

13. Anticorps destiné à être utilisé selon la revendication 1, dans lequel l'anticorps est un Fab, un Fab', un F(ab')2, un F(ab)c, ou un Fv.
